# EUROPÄISCHE PATENTSCHRIFT

(11) **EP 0 578 224 B1**
(45) Veröffentlichungstag und Bekanntmachung des Hinweises auf die Patenterteilung: **07.05.1997**
(21) Anmeldenummer: 93110861.7
(22) Anmeldetag: 07.07.1993
(51) Int. Cl.: A61G 13/10

(54) **Operationstischplatte**
Operating platform
Support d'opération

(30) Priorität: 10.07.1992 DE 4222639
(43) Veröffentlichungstag der Anmeldung: 12.01.1994
(73) Patentinhaber: Blanco GmbH & Co. KG, D-75038 Oberderdingen (DE)
(72) Erfinder: Dietrich, Uwe, D-55767 Schwollen (DE); Gass, René, D-76646 Bruchsal-Heidelstein (DE); Lerrahn, Wilfried, D-75056 Sulzfeld (DE); Nagel, Hartmut, D-07407 Rudolstadt-Planzwirbach (DE); Ruppert, Peter, D-76646 Bruchsal (DE); Stolze, Dirk, D-07318 Saalfeld (DE); Zanin, Gian-Pietro, D-75015 Bretten (DE)
(74) Vertreter: Hoeger, Stellrecht & Partner

(56) Entgegenhaltungen:
- DE-U- 9 114 295
- GB-A- 2 095 545
- GB-A- 2 230 948

## Beschreibung

Die Erfindung betrifft eine Operationstischplatte mit einer Polsterauflage, wie sie im Oberbegriff des Anspruchs 1 beschrieben ist.

Operationstischplatten dieser Art sind aus dem Prospekt "Blanco Operationstisch BMT 300" bekannt. Sie werden sowohl zur Lagerung eines Patienten während eines operativen Eingriffs als auch zum Transport eines Patienten, beispielsweise auf dem Weg vom Operationssaal zur Röntgenabteilung eines Krankenhauses verwendet. Dabei kann die Operationstischplatte für den Transport vom Rest des Operationstisches getrennt und auf einen Transportwagen montiert werden. Mit weiteren Zugspindel-Aggregaten und Stützen versehen, kann die Operationstischplatte auch als Extensions-Tischplatte verwendet werden.

Für die operationsgerechte Lagerung eines Patienten können einzelne Segmente der Operationstischplatte gegeneinander verschwenkt und/oder verschoben werden. So wird zum Beispiel bei einer Leberoperation der Zugang zur Leber des Patienten vereinfacht, indem der Patient in Seitenlage gebracht wird und die am Hüft- beziehungsweise am Brustbereich des Patienten anliegenden Segmente der Operationstischplatte gegeneinander verkippt werden, so daß sich ein Körperbogen ausbildet.

Bei Operationstischplatten der vorbekannten Art befindet sich auf der Tischplatte ein Polster, welches auf seiner ganzen Länge fest mit der Operationstischplatte verbunden ist. Dabei ist das Polster in einzelne Platten unterteilt, die den Segmenten der Operationstischplatte entsprechen. Die einzelnen Polsterplatten sind im Rücken- und im Beckenbereich des Patienten auf ihrer Oberseite über einen dünnen Bezug miteinander verbunden. Werden bei diesen Operationstischplatten einzelne Segmente gegeneinander verkippt oder verschwenkt, so verändert sich dadurch der relative Abstand zwischen den Polsterplatten in Längsrichtung. Bei einem auf der Operationstischplatte liegenden Patienten wird somit beim Herstellen einer operationsgerechten Lagerung Gewebe gestreckt oder gestaucht und eventuell zwischen den Polsterplatten eingequetscht. Durch diese Strekkung beziehungsweise Quetschung kann die Gewebedurchblutung des Patienten stark eingeschränkt oder gar vollständig unterbrochen werden. Dies kann vor allem bei länger andauernden Operationen zu sehr schmerzhaften und nur langsam ausheilenden Nekrosen führen.

Aus der DE 40 14 354 A1 ist eine Stützfläche bekannt, die sich insbesondere für die Verwendung in Krankenhausmöbeln, als Patientenstützflächen für Unfallopfer sowie für Möbelstücke für die Gesundpflege eignet. Die Stützfläche ist in Längsrichtung wenigstens in zwei im wesentlichen starre Stützelemente unterteilt, die miteinander durch ein flexibles Verbindungselement so verbunden sind, daß eine Relativbewegung zwischen den Stützelementen erfolgen kann. Im Idealfall ist die Stützfläche in vier Stützelemente unterteilt, die so verstellbar sind, daß sie entweder nur eine Rückenlehne oder eine Stuhlform mit angepaßter Kontur bilden. Die flexiblen Verbindungselemente ermöglichen einen gekrümmten Übergang zwischen zwei benachbarten Stützelementen. Zur Abstützung eines Patienten ist auf der Stützfläche eine einteilige Matratze positioniert, die an ihren Längsseitenwänden Falten aufweist, um eine größere Flexibilität zu erzielen. Wird die Stützfläche durch Schwenken der Stützelemente beispielsweise in eine Stuhlform gebracht, so verändert sich dadurch der relative Abstand zwischen bestimmten Bereichen der Matratze in Längsrichtung; dies kann wiederum dazu führen, daß bei einem auf der Matratze liegenden Patienten Gewebe gestreckt oder gestaucht wird.

Aus der DE 91 14 295 U1 ist eine Untersuchungsliege mit einem gepolsterten Abstützteil bekannt, der in Längsrichtung in einen feststehenden Bereich und einen schwenkbaren Bereich unterteilt ist. Der Abstützteil ist mit Hilfe einer aus Zapfen und korrespondierenden Buchsen bestehenden Verbindungskonstruktion lösbar an einem den Abstützteil tragenden Gestell verbunden. Die beiden Bereiche des Abstützteiles weisen eine in üblicher Weise gepolsterte Oberseite auf, damit ein Patient bequem gelagert werden kann. Wird der schwenkbare Bereich gegen den feststehenden Bereich verschwenkt, so ist damit bei einem auf der Untersuchungsliege positionierten Patienten eine Stauchung oder Streckung von Gewebe verbunden, und evtl. wird Gewebe zwischen dem feststehenden Bereich und dem schwenkbaren Bereich eingequetscht. Wie bereits oben beschrieben, kann eine derartige Streckung bzw. Quetschung von Gewebepartien zu einer Einschränkung oder gar zu einer vollständigen Unterbrechung der Gewebedurchblutung des Patienten führen.

Aufgabe der Erfindung ist es, beim Schwenken und/oder Verschieben benachbarter Segmente der Operationstischplatte zur Herstellung einer operationsgerechten Lagerung des Patienten eine Streckung oder Stauchung von Gewebepartien des Patienten zu vermeiden.

Diese Aufgabe wird bei einer Operationstischplatte der eingangs beschriebenen Art erfindungsgemäß dadurch gelöst, daß die Polsterauflage einteilig geformt ist und daß die Unterseite der Polsterauflage an einem Segment der Operationstischplatte ortsfest gehalten und gegenüber einem weiteren Segment in Längsrichtung der Operationstischplatte verschieblich gelagert ist.

Durch die einteilige Form der Polsterauflage ist eine Veränderung des relativen Abstands einzelner Bereiche der Polsterauflage beim Schwenken und/oder Verschieben der Segmente der Operationstischplatte ausgeschlossen. Statt dessen verändert sich beim Schwenken und/oder Verschieben der Segmente die Lage der verschieblich gelagerten Unterseite der Polsterauflage relativ zur Operationstischplatte. Da die Auflagefläche für den Patienten weder gestreckt noch gestaucht wird, kommt es beim Herstellen einer operationsgerechten Lagerung des Patienten auch nicht zu einer Streckung oder Quetschung von Gewebepartien des Patienten und einer damit verbundenen mangelnden Gewebedurchblutung. Selbst bei sehr lang andauernden Operationen ist damit eine Nekrosenbildung aufgrund von Streckungen oder Stauchungen ausgeschlossen.

Bei einer besonderen Ausführungsform der Operationstischplatte kann vorgesehen sein, daß die Operationstischplatte in zwei Segmente unterteilt ist, die den Rückenbereich beziehungsweise den Beckenbereich des Patienten aufnehmen. Gegebenenfalls sind an die Operationstischplatte zur Unterstützung des Kopfes und der Beine des Patienten weitere Segmente oder spezielle Haltevorrichtungen, beispielsweise verstellbare Beinhalter, ansetzbar. Diese Operationstischplatte kann beispielsweise in der Urologie für endovesikale Eingriffe verwendet werden.

Günstig ist es, wenn die Polsterauflage der Operationstischplatte im Bereich des den Schwerpunkt des Patienten unterstützenden Segments mit der Operationstischplatte ortsfest gehalten ist. Dadurch bleibt das Segment der Operationstischplatte, welches den größten Anteil der Gewichtskraft des Patienten aufzunehmen hat, unbewegt. Die entsprechend geringeren Anteile der Gewichtskraft, die von den verschieblich gelagerten Segmenten aufzunehmen sind, bewirken beim Verschieben der Segmente eine entsprechend geringere Reibungskraft zwischen der Unterseite der Polsterauflage und der Operationstischplatte. Dadurch erleichtert sich das Schwenken und/oder Verschieben der Segmente zur Herstellung einer operationsgerechten Lagerung des Patienten.

In einer besonderen Ausführungsform kann vorgesehen sein, daß die Polsterauflage lösbar mit der Operationstischplatte verbindbar ist. Die ortsfeste Halterung der Polsterauflage an der Operationstischplatte kann beispielsweise über Druckknöpfe oder Klettverschlüsse erfolgen. Besonders günstig ist es, wenn die Verbindungselemente zwischen der Unterseite der Polsterauflage und der Operationstischplatte am Rand der Polsterauflage angeordnet sind. Dadurch ergibt sich ein einfacher Zugang zu den Verbindungselementen und eine einfache Handhabung derselben.

Es kann auch vorgesehen sein, daß eine durchgängige Lage, beispielsweise die Oberseite, der Polsterauflage in Längsrichtung einen solchen Elastizitätsmodul aufweist, daß die aus der durchschnittlichen Reibungskraft zwischen der Polsterauflage und der Operationstischplatte resultierende Zugkraft allenfalls eine vernachlässigbare Längsdehnung der Polsterauflage bewirkt. Beim Schwenken und/oder Verschieben der Segmente bewirkt die Gewichtskraft des Patienten eine Reibungskraft zwischen der Polsterauflage und der Operationstischplatte. Daraus resultiert eine auf die Polsterauflage einwirkende Zugkraft, die abhängig von der Größe des Elastizitätsmoduls der durchgängigen Lage, beispielsweise der Oberseite, der Polsterauflage eine Längsdehnung derselben bewirkt. Da mit der Längsdehnung der Polsterauflage beziehungsweise der Entspannung aus dem gedehnten Zustand eine Dehnung beziehungsweise Stauchung von Gewebepartien des Patienten verbunden ist, muß der Elastizitätsmodul so gewählt werden, daß die Längsdehnung der Oberseite der Polsterauflage wenige Prozentpunkte, beispielsweise weniger als 5% beträgt, so daß die Blutversorgung des gestauchten oder gestreckten Gewebes des Patienten nur unwesentlich beeinflußt und die Versorgung der Gewebezellen mit Nährstoffen jederzeit gewährleistet ist.

In einer weiteren Ausführungsform der Operationstischplatte kann vorgesehen sein, daß an der Unterseite der Polsterauflage und an der ihr gegenüberliegenden Seite der Operationstischplatte zusammenwirkende Führungselemente, insbesondere komplementär ausgebildete oder ineinandergreifende Nuten beziehungsweise Rippen in Längsrichtung angeordnet sind. Diese Führungselemente lagern die Polsterauflage an den verschieblichen Segmenten an der Operationstischplatte. Die Führungselemente können außerdem eine verbesserte Stabilität der Polsterauflage in Längsrichtung und in Querrichtung bewirken. Zur Steigerung der Formstabilität und zur Versteifung der Polsterauflage können weitere mechanische Elemente an der Polsterauflage angeordnet sein. Es kann auch vorgesehen sein, daß die Polsterauflage aus Kunststoff gefertigt ist und die Makromoleküle des Kunststoffs bereichsweise zusätzlich stark vernetzt werden, dabei ihre Elastizität teilweise verlieren und zur Versteifung der Polsterauflage beitragen. Eine Ausbildung der zusammenwirkenden Führungselemente zwischen der Polsterauflage und der Operationstischplatte in Form von ineinandergreifenden Nuten beziehungsweise Rippen hat den Vorteil, daß die Montage der Polsterauflage auf der Operationstischplatte sehr einfach erfolgen kann. Dazu müssen die beispielweise an der Unterseite der Polsterauflage angeordneten Rippen an die auf der Oberseite der Operationstischplatte angeordneten Nuten angesetzt und ineinander geschoben werden und nach der vollständigen Einführung zur ortsfesten Halterung der Polsterauflage an einem Segment der Operationstischplatte die Halteelemente, beispielsweise die Druckknöpfe, betätigt werden.

Bei einer anderen Ausgestaltung der Operationstischplatte kann vorgesehen sein, daß ein endständiges Segment der Operationstischplatte und der entsprechende Bereich der Polsterauflage parallel zur Längsrichtung, in bezug auf die Querrichtung mittig geteilt sind. Der dem endständigen Segment der Operationstischplatte entsprechende Bereich der Polsterauflage kann beispielsweise zur Aufnahme der Beine des Patienten verwendet werden. Die Teilung des Segments der Operationstischplatte und der Polsterauflage parallel zur Längsrichtung ermöglicht dann eine unterschiedliche Lagerung der Beine des Patienten. Beispielsweise kann für eine Unterschenkeloperation eine Beinplatte nach unten geschwenkt werden, während die andere Beinplatte horizontal ausgerichtet bleibt.

Bei einer bevorzugten Ausführungsform der Operationstischplatte ist die Polsterauflage aus einem eine hohe Transparenz für Röntgenstrahlung aufweisenden Material gefertigt. Dadurch wird eine röntgenologische Untersuchung eines auf der Operationstischplatte liegenden Patienten ermöglicht, ohne den Patienten von der Operationstischplatte abnehmen zu müssen.

In einer weiteren bevorzugten Ausführungsform der Operationstischplatte ist die Polsterauflage mit einem elektrisch leitenden Bezug überzogen, wodurch eine elektrostatische Aufladung der Polsterauflage, beispielsweise durch Reibungselektrizität, vermieden wird.

Die nachfolgende Beschreibung bevorzugter Ausführungsformen der Erfindung dient im Zusammenhang mit der Zeichnung der näheren Erläuterung.

Es zeigen:
- Fig. 1: eine schaubildliche Ansicht eines Operationstisches mit einer in zwei Segmente unterteilten Operationstischplatte, einer einteiligen Polsterauflage und mit einer angesetzten Beinplatte;
- Fig. 2: eine schaubildliche Ansicht eines Operationstisches mit einer in drei Segmente unterteilten Operationstischplatte und einer einteiligen Polsterauflage, wobei das die Beine des Patienten aufnehmende, endständige Segment der Tischplatte und der entsprechende Bereich der Polsterauflage parallel zur Längsrichtung, in bezug auf die Querrichtung mittig geteilt sind;
- Fig. 3: eine Teilschnittansicht der Operationstischplatte längs der Linie 3-3 in Figur 1;
- Fig. 4: eine Draufsicht der Operationstischplatte in Richtung des Pfeiles A in Figur 3 und
- Fig. 5: eine Schnittansicht der Operationstischplatte längs der Linie 5-5 in Figur 1.

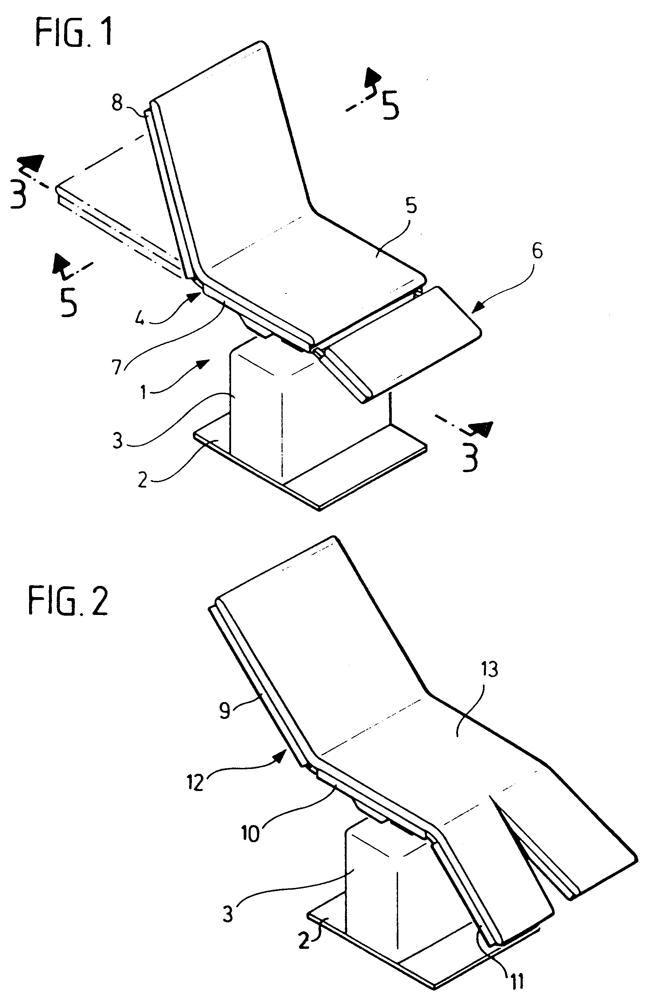

Figur 1 zeigt einen Operationstisch 1, welcher eine Grundplatte 2 und eine auf der Grundplatte angeordnete Säule 3 umfaßt, sowie eine auf der Säule befestigte Operationstischplatte 4 mit einer Polsterauflage 5. An die Operationstischplatte 4 ist zusätzlich eine Beinplatte 6 angesetzt. Die Operationstischplatte 4 ist in ein Sitzsegment 7 und ein Rückensegment 8 unterteilt. Ausgehend von einer in Figur 1 strichpunktiert dargestellten horizontalen Lage des Rückensegments 8, kann dieses sowohl nach oben, wie in Figur 1 in durchgezogener Linie gezeigt, als auch nach unten geschwenkt werden (nicht dargestellt).

Figur 2 zeigt als weitere Ausführungsform eine in ein Rückensegment 9, ein Sitzsegment 10 und ein Beinsegment 11 unterteilte Operationstischplatte 12. Die Operationstischplatte 12 kann alternativ zur Operationstischplatte 4 (Fig. 1) auf der Säule 3 montiert werden. Eine einteilige Polsterauflage 13 bedeckt die Operationstischplatte 12 vollständig. Das Beinsegment 11 der Operationstischplatte 12 ist ebenso wie der entsprechende Bereich der Polsterauflage 13 parallel zur Längsrichtung in bezug auf die Querrichtung mittig geteilt. Die Unterteilung des Beinsegments 11 und des entsprechenden Bereiches der Polsterauflage 13 ermöglicht eine unterschiedliche Lagerungsposition der Beine der Patienten.

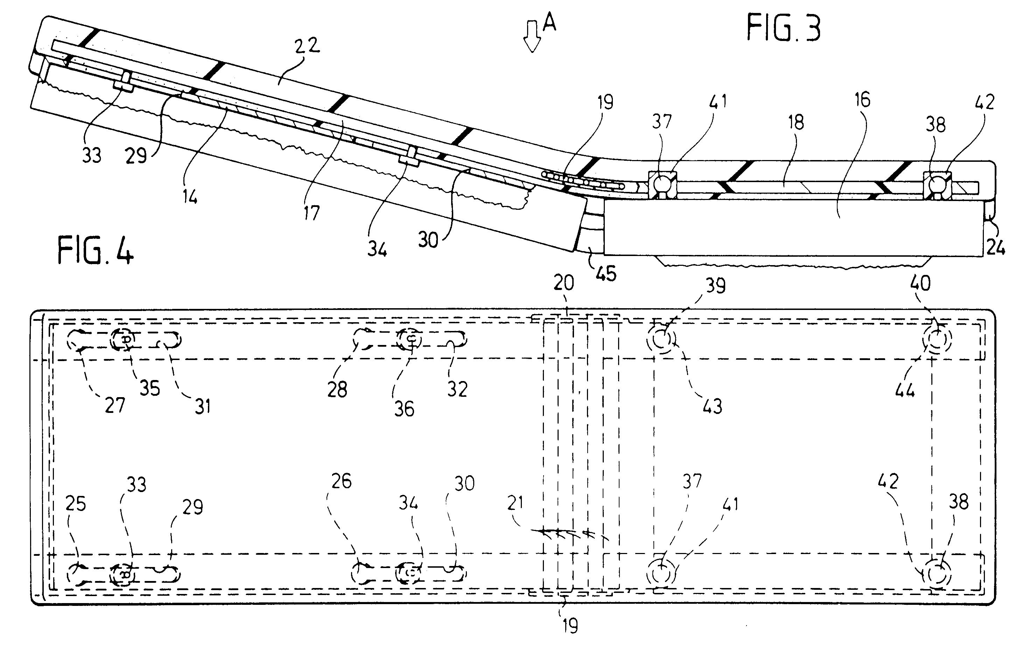

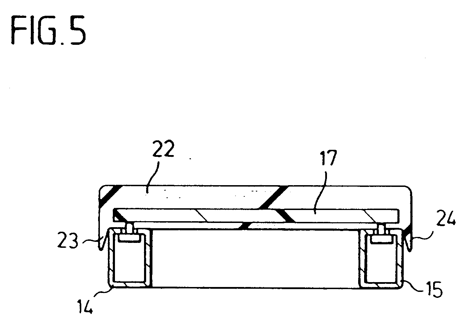

Wie am besten aus Figur 5 und Figur 3 hervorgeht, umfaßt das Rückensegment 8 zwei in Längsrichtung am Rand der Operationstischplatte 4 angeordnete, ein Hohlprofil aufweisende Holmen 14, 15. Über ein in Figur 3 schematisch dargestelltes Gelenk 45 schließt sich an den Holm 14 des Rückensegmentes 8 ein Holm 16 des Sitzsegmentes 7 der Operationstischplatte 4 an. Entsprechend ist über ein Gelenk ein in der Zeichnung nicht gezeigter zweiter Holm des Sitzsegmentes 7 mit dem Holm 15 des Rückensegments 8 verbunden.

Die Polsterauflage 5 umfaßt zwei rechteckige, dem Sitzsegment 7 bzw. dem Rückensegment 8 entsprechende Hartstoffplatten 17, 18. Die beiden Hartstoffplatten 17, 18 sind über parallel zur Längsrichtung, jeweils an einem Rand der Operationstischplatte 4 angeordnete, identische Ketten 19, 20 miteinander verbunden. Zur Erhöhung der Formstabilität der Polsterauflage sind die sich jeweils entsprechenden Kettenglieder der beiden Ketten über senkrecht zur Längsrichtung der Operationstischplatte 4 angeordnete Verbindungsstangen 21 starr miteinander verbunden. Die Hartstoffplatten 17, 18 sind ebenso wie die Ketten 19, 20 und die Verbindungsstangen 21 vollständig von einem elastischen Schaumstoff 22 umgeben. Durch den Schaumstoff 22 erhält die Polsterauflage 5 eine einteilige, rechteckige, den Außenkonturen der Operationstischplatte 4 entsprechende Form. Zusätzlich bildet der Schaumstoff 22 entlang der Längsseiten der Operationstischplatte 4 die Holmen des Sitzsegments 7 und des Rückensegments 8 auf der jeweiligen Außenseite teilweise überdeckende Abtropflappen 23, 24.

Eine wesentliche Maßnahme gemäß der Erfindung stellen die Verbindungselemente zwischen der Polsterauflage 5 und der Operationstischplatte 4 dar. In den der Polsterauflage gegenüberliegenden Oberseiten der Holmen 14, 15 des Rücksegments 8 sind im Abstand von etwa der halben Längsausdehnung der Holmen jeweils zwei Bohrungen 25, 26 beziehungsweise 27, 28 eingebracht. An die einzelnen Bohrungen schließt sich jeweils ein Langloch 29, 30 beziehungsweise 31, 32 an mit geringerem Durchmesser als der Durchmesser der Bohrungen. In die den Holmen 14, 15 des Rückensegments 8 gegenüberliegende Unterseite der Hartstoffplatte 17 sind ein T-förmiges Profil aufweisende Stifte 33, 34 beziehungsweise 35, 36 so angeordnet, daß bei horizontaler Lagerung des Rückensegments 8 die Position der Stifte jeweils mit der Mitte eines gegenüberliegenden Langloches übereinstimmt. Zur Befestigung der Polsterauflage 5 am Rückensegment 8 der Operationstischplatte 4 wird die Polsterauflage 5 so auf das horizontal ausgerichtete Rückensegment 8 aufgelegt, daß die Position der Stifte 33, 34, 35, 36 mit den jeweiligen Bohrungen 25, 26, 27 ,28 übereinstimmt. Die Stifte werden in die Bohrungen eingeführt und die Polsterauflage anschließend in Richtung auf das Sitzsegment 7 der Operationstischplatte 4 verschoben. Dadurch gelangen die Stifte in den Bereich der Länglöcher und da der Außendurchmesser der Stiftköpfe größer ist als die Breite der Langlöcher, kann die Polsterauflage nicht mehr senkrecht abgehoben werden. Gleichzeitig garantieren die Langlöcher, daß die Polsterauflage 5 verschieblich auf dem Rückensegment 8 gelagert ist. Die Länge der Langlöcher bestimmt den möglichen Verschiebeweg der Polsterauflage auf den Holmen und damit der Auslenkwinkel des Rückensegmentes aus der Horizontalrichtung.

An den der Polsterauflage 5 gegenüberliegenden Oberseiten der Holmen des Sitzsegmentes 7 sind, jeweils einer Stirnfläche der Holmen benachbart, nach oben weisende Noppen 37, 38 beziehungsweise 39, 40 angeordnet. Bei aufgesetzter Polsterauflage greifen die Noppen in in der Hartstoffplatte 18 der Polsterauflage 5 eingefügte Hülsen 41, 42 beziehungsweise 43, 44, die eine komplementär zur Außenfläche der Noppen ausgebildete Innenfläche aufweisen. Die Hülsen sind aus einem elastischen Material gefertigt, so daß sie über die Noppen übergestülpt werden können. Noppen und Hülsen bilden somit eine ortsfeste Halterung des Sitzsegments 10 der Polsterauflage 5.

## Patentansprüche

1. Operationstischplatte (4) mit einer Polsterauflage (5), deren Oberseite als Auflagefläche für einen Patienten ausgebildet ist und deren Unterseite mit der Operationstischplatte (4) verbunden ist, wobei die Operationstischplatte (4) in Längsrichtung in mehrere gegeneinander schwenk- und/oder verschiebbare Segmente (8,7;9,10,11) unterteilt ist und wobei die Polsterauflage (5) die Liegefläche der Tischplatte im wesentlichen vollständig abdeckt,
dadurch gekennzeichnet, daß
daß die Polsterauflage (5) einteilig geformt ist und daß die Unterseite der Polsterauflage (5) an einem Segment (7;10) der Operationstischplatte (4) ortsfest gehalten und gegenüber einem weiteren Segment (8;9) in Längsrichtung der Operationstischplatte (4) verschieblich gelagert ist.

2. Operationstischplatte nach Anspruch 1, dadurch gekennzeichnet, daß die Operationstischplatte (4) in zwei den Rückenbereich beziehungsweise den Beckenbereich des Patienten aufnehmende Segmente (8,7;9,10) unterteilt ist und daß gegebenenfalls an die Operationstischplatte (4) weitere Segmente (6) zur Unterstützung des Kopfes und der Beine des Patienten ansetzbar sind.

3. Operationstischplatte nach Anspruch 1 oder 2, dadurch gekennzeichnet, daß die Polsterauflage (5) im Bereich des den Schwerpunkt des Patienten unterstützenden Segments (7;10) mit der Operationstischplatte (4) ortsfest gehalten ist.

4. Operationstischplatte nach einem der vorgenannten Ansprüche, dadurch gekennzeichnet, daß die Polsterauflage (5) lösbar mit der Operationstischplatte (4) verbindbar ist.

5. Operationstischplatte nach einem der vorgenannten Ansprüche, dadurch gekennzeichnet, daß die Polsterauflage (5) eine sich im wesentlichen über die gesamte Länge der Polsterauflage erstreckende Lage umfaßt, insbesondere die Oberseite der Polsterauflage, welche in Längsrichtung einen solchen Elastizitätsmodul aufweist, daß die aus der durchschnittlichen Reibungskraft zwischen Polsterauflage (5) und Operationstischplatte (4) resultierende, auf die Polsterauflage (5) wirkende Zugkraft höchstens eine vernachlässigbare Längsdehnung der Polsterauflage (5) bewirkt.

6. Operationstischplatte nach einem der vorgenannten Ansprüche, dadurch gekennzeichnet, daß an der Unterseite der Polsterauflage (5) und an der ihr gegenüberliegenden Seite der Operationstischplatte (4) zusammenwirkende Führungselemente, insbesondere komplementär ausgebildete oder ineinandergreifende Nuten beziehungsweise Rippen, in Längsrichtung angeordnet sind.

7. Operationstischplatte nach einem der vorgenannten Ansprüche, dadurch gekennzeichnet, daß ein endständiges Segment (11) der Operationstischplatte (4) und der entsprechende Bereich der Polsterauflage (5) parallel zur Längsrichtung, in bezug auf die Querrichtung mittig geteilt sind.

8. Operationstischplatte nach einem der vorgenannten Ansprüche, dadurch gekennzeichnet, daß die Polsterauflage (5) aus einem eine hohe Transparenz für Röntgenstrahlen aufweisenden Material gefertigt ist.

9. Operationstischplatte nach einem der vorgenannten Ansprüche, dadurch gekennzeichnet, daß die Polsterauflage (5) mit einem elektrisch leitenden Bezug überzogen ist.

## Claims

1. An operating table top (4) having a padded covering (5), the upper surface of which is formed as a supporting surface for a patient and the underside of which is connected to the operating table top (4), wherein the operating table top (4) is divided in the longitudinal direction into a plurality of segments (8, 7; 9, 10, 11) pivotable and/or displaceable relative to one another and wherein the padded covering (5) substantially completely covers the supporting surface of the table, characterised in that the padded covering (5) is formed in one piece and in that the underside of the padded covering (5) is fixedly held on one segment (7; 10) of the operating table top (4) and is movably mounted relative to another segment (8; 9) in the longitudinal direction of the operating table top (4).

2. An operating table top according to claim 1, characterised in that the operating table top (4) is divided into two segments (8, 7; 9, 10) supporting the back region and the pelvic region of the patient and in that further segments (6) are optionally attachable to the operating table top (4) for supporting the head and the legs of the patient.

3. An operating table top according to claim 1 or 2, characterised in that the padded covering (5) is fixedly connected to the operating table top (4) in the region of the segment (7; 10) supporting the centre of gravity of the patient.

4. An operating table top according to any of the preceding claims, characterised in that the padded covering (5) is detachably connectable to the operating table top (4).

5. An operating table top according to any one of the preceding claims, characterised in that the padded covering (5) comprises a layer extending substantially over the entire length of the padded covering, in particular the upper surface of the padded covering, and, in the longitudinal direction, having an elasticity modulus such that the tensile force resulting from the average frictional force between the padded covering (5) and the operating table top (4) and acting on the padded covering (5) causes at most a negligeable elongation of the padded covering (5).

6. An operating table top according to any one of the preceding claims, characterised in that co-operating guide members, in particular complementary or mutually engaging grooves and ribs, are arranged in the longitudinal direction on the underside of the. padded covering (5) and on the side of the operating table top (4) facing the padded covering (5).

7. An operating table top according to any one of the preceding claims, characterised in that an end segment (11) of the operating table top (4) and the corresponding region of the padded covering (5) are divided parallel to the longitudinal direction and centrally relative to the transverse direction.

8. An operating table top according to any one of the preceding claims, characterised in that the padded covering (5) is made from a material with high transmittance for X-rays.

9. An operating table top according to any one of the preceding claims, characterised in that the padded covering (5) is covered with an electrically conductive coating.

## Revendications

1. Plateau (4) de table d'opération comportant un support rembourré (5), dont le côté supérieur sert de surface d'appui pour un patient et dont le côté inférieur est relié au plateau (4) de la table d'opération, ce plateau (4) de la table d'opération étant divisé dans la direction longitudinale en plusieurs segments (8, 7 ; 9, 10, 11), déplaçables relativement par pivotement et/ou par translation, le support rembourré (5) recouvrant sensiblement complètement la surface d'appui du plateau de table, caractérisé en ce que le support rembourré (5) est formé unitairement et en ce que le côté inférieur de ce support rembourré (5) est maintenu en position fixe sur un segment (7 ; 10) du plateau (4) de la table d'opération et est disposé par rapport à un autre segment (8 ; 9) avec possibilité de translation dans la direction longitudinale du plateau (4) de la table d'opération.

2. Plateau de table d'opération selon la revendication 1, caractérisé en ce que le plateau (4) de la table d'opération est divisé en deux segments (8, 7 ; 9, 10) recevant la zone dorsale ou la zone de bassin du patient et en ce que le cas échéant d'autres segments (6) servant à soutenir la tête et les jambes du patient peuvent être reliés au plateau (4) de la table d'opération.

3. Plateau de table d'opération selon une des revendications 1 ou 2, caractérisé en ce que le support rembourré (5) est maintenu fixé avec le plateau (4) de la table d'opération dans une zone du segment (7 ; 10) soutenant le centre de gravité du patient.

4. Plateau de table d'opération selon une des revendications précédentes, caractérisé en ce que le support rembourré (5) est relié de façon séparable au plateau (4) de la table d'opération.

5. Plateau de table d'opération selon une revendications précédentes, caractérisé en ce que le support rembourré (5) comporte une surface s'étendant dans l'essentiel sur toute la longueur du support rembourré (5), notamment le côté supérieur du support rembourré et possédant dans la direction longitudinale, un module d'élasticité qui soit tel que la force de traction s'exerçant sur le support rembourré (5) et résultant de la force moyenne de frottement engendrée entre le support rembourré (5) et le plateau (4) de la table d'opération produise au maximum un allongement longitudinal négligeable du support rembourré (5).

6. Plateau de table d'opération selon une des revendications précédentes, caractérisé en ce que des éléments de guidage, coopérant entre eux sur le côté inférieur du support rembourré (5) et sur le côté correspondant du plateau (4) de la table d'opération, notamment des nervures et des rainures de profil complémentaires ou à emboîtement mutuel, sont disposées dans la direction longitudinale.

7. Plateau de table d'opération selon une des revendications précédentes, caractérisé en ce qu'un segment extrême (11) du plateau (4) de la table d'opération et la zone correspondante du support rembourré (5) sont divisés parallèlement à la direction longitudinale et au milieu par rapport à la direction transversale.

8. Plateau de table d'opération selon une des revendications précédentes, caractérisé en ce que le support rembourré (5) est réalisé en un matériau ayant une grande transparence aux rayons X.

9. Plateau de table d'opération selon une des revendications précédentes, caractérisé en ce que le support rembourré (5) est recouvert d'un revêtement électriquement conducteur.
